# FASCICULE DE BREVET EUROPEEN

(11) **EP 1 651 668 B1**
(45) Date de publication et mention de la délivrance du brevet: **15.07.2009**
(21) Numéro de dépôt: 04786256.0
(22) Date de dépôt: 04.08.2004
(51) Int. Cl.: C07K 14/47

(54) **NOUVEAU PEPTIDE INTERAGISSANT AVEC LES PROTEINES ANTI-APOPTOTIQUES DE LA FAMILLE BCL-2**
PEPTID WELCHES MIT DEN ANTI-APOPTOTISCHEN PROTEINEN DER FAMILIE BCL-2 INTERAGIERT
NOVEL PEPTIDE INTERACTING WITH ANTI-APOPTOTIC PROTEINS OF A BCL-2 FAMILY

(30) Priorité: 06.08.2003 FR 0309697
(43) Date de publication de la demande: 03.05.2006
(73) Titulaire: Les Laboratoires Servier, 92415 Courbevoie Cedex (FR); Hybrigenics, 75014 Paris (FR)
(72) Inventeur: GENESTE, Olivier, F-92500 Rueil-Malmaison (FR); HICKMAN, John, F-75017 Paris (FR); BENNETT, Richard, F-75015 Paris (FR); RAIN, Jean- Christophe, F-95120 Ermont (FR)
(86) Numéro de dépôt international: PCT/FR2004/002081
(87) Numéro de publication internationale: WO 2005/014638

(56) Documents cités:
- US-A- 5 283 173
- DATABASE GENESEQ [Online] 6 mars 2003 (2003-03-06), "Human Bcl-XL-binding polypeptide" XP002268229 Database accession no. ABJ18850 -& WO 02/072761 A (PHYLOS INC) 19 septembre 2002 (2002-09-19)
- KELEKAR AMEETA ET AL: "Bcl-2-family proteins: The role of the BH3 domain in apoptosis" TRENDS IN CELL BIOLOGY, vol. 8, no. 8, août 1998 (1998-08), pages 324-330, XP002268228 ISSN: 0962-8924
- DEGTEREV A ET AL: "IDENTIFICATION OF SMALL-MOLECULE INHIBITORS OF INTERACTION BETWEEN THE BH3 DOMAIN AND BCL-XL" NATURE CELL BIOLOGY, MACMILLAN PUBLISHERS, GB, vol. 3, février 2001 (2001-02), pages 173-182, XP008000872 ISSN: 1465-7392
- OWICKI J C: "FLUORESCENCE POLARIZATION AND ANISOTROPY IN HIGH THROUGPUT SCREENING: PERSPECTIVES AND PRIMER" JOURNAL OF BIOMOLECULAR SCREENING, LARCHMONT, NY, US, vol. 5, no. 5, octobre 2000 (2000-10), pages 297-306, XP009007612 ISSN: 1087-0571 cité dans la demande

## Description

La présente invention concerne un nouveau peptide interagissant avec Bcl-XL et/ou Bcl-2 et/ou Bcl-W, ainsi que les méthodes de criblage permettant d'identifier des molécules capables de moduler cette interaction.

La plupart des processus biologiques impliquent des interactions protéines-protéines. Un des objectifs fixés par la protéomique est la réalisation d'une carte de ces interactions. Celles-ci, impliquées dans la plupart des mécanismes de transductions de signal, sont des cibles de choix dans l'élaboration d'un médicament.

Il existe de nombreuses méthodologies permettant d'identifier des interactions protéiques. Une des plus répandues est le système du double hybride initialement développée et décrite par Fields et al. (US 5,283,173; US 5,468,614; US 5,667,973).
Ce système consiste à la base en un test *in vitro* entre deux protéines recombinées. La première appelée protéine « appât » est une protéine chimérique fusionnée à un domaine de liaison de l'ADN (DNA binding domain/BD) capable de se lier en amont d'un gène reporter. Les domaines de liaison couramment utilisés sont ceux de Gal4 ou *E.coli* LexA.
La seconde protéine est également une protéine chimérique communément appelée « proie » qui contient un domaine d'activation (activation domain/AD), en général provenant de Gal4. Cependant, ces méthodes conventionnelles ont leurs limites. Il est par exemple bien connu que de tels criblages peuvent conduire à des faux positifs et/ou faux négatifs et des confirmations biochimiques des résultats obtenus sont nécessaires.
Une technique plus performante permettant de minimiser les faux positifs ou négatifs est décrite dans la demande de brevet WO9942612 et utilise des levures haploïdes recombinantes contenant les protéines « appât » et « proie ». Ce système permet la détection d'un plus grand nombre de « proies » à partir d'un « appât », de façon plus précise, plus reproductible et plus sensible que les autres méthodes conventionnelles utilisées dans le domaine.

L'apoptose est un processus de mort cellulaire jouant un rôle crucial chez les organismes pluricellulaires. Il existe en effet deux formes de mort cellulaire : la nécrose et l'apoptose. La nécrose se rencontre lors de lésions tissulaires : les cellules gonflent, conduisant à la fuite des constituants cellulaires puis à la lyse de la cellule, ce qui provoque une inflammation des tissus alentours.
Au contraire, l'apoptose est un processus physiologique programmé et régulé, dont on ne peut sous estimer l'importance puisque environ 10⁹ de nos cellules meurent tous les jours par ce mécanisme. De nombreuses pathologies sont reliées à une dérégulation de l'équilibre existant entre la croissance, la survie et la mort cellulaire.
On peut citer en particulier les maladies autoimmunes, certaines maladies neurologiques et les cancers.
Maintenir une cellule en vie ou programmer sa mort nécessite au moins dix familles de protéines différentes, parmi lesquelles la famille Bcl-2 joue un rôle majeur. Cette famille contient environ vingt protéines parmi lesquelles Bcl-2, Bcl-XL et Bcl-W, protéines anti-apoptotiques favorisant la survie de la cellule, à l'inverse de Bax, Bak et Bid qui sont des protéines pro-apoptotiques. Au cours de l'apoptose, il semblerait que les membres de la famille Bcl-2 modifient leurs interactions avec leurs partenaires de façon à induire des changements irréversibles dans la cellule conduisant à la mort cellulaire.

Il est ainsi essentiel de pouvoir identifier des molécules capables de modifier ces interactions pour obtenir de réels candidats médicaments efficaces dans les pathologies impliquant des dérégulations de l'apoptose, notamment les maladies autoimmunes, certaines maladies neurodégénératives et les cancers.

La demanderesse a présentement identifié un nouveau peptide interagissant avec les protéines anti-apoptotiques de la famille Bcl-2, et plus particulièrement Bcl-2, Bcl-XL et Bcl-W.

Ce peptide de 22 acides aminés correspond au domaine précis d'interaction avec les protéines anti-apoptotiques de la famille Bcl-2 et plus particulièrement Bcl-2 et/ou Bcl-XL et/ou Bcl-W, et possède les critères structuraux typiques d'un motif « BH3 », domaine d'interaction permettant la formation d'homo- ou d'hétérodimères.

La faible taille de ce peptide en fait un candidat idéal pour l'élaboration d'un test permettant le criblage hautement efficace de molécules capables de moduler les interactions entre ces protéines.

On trouve dans la littérature de nombreux tests de criblage de modulateurs d'interactions protéines-protéines mais ils présentent souvent des limites quant à leur sensibilité et leur faisabilité à haut débit. Les méthodes couramment utilisées nécessitent la mise en oeuvre d'outils complexes (interactions entre protéines de fusion, entre protéines recombinantes...) peu compatible avec un criblage à haut débit. Elles génèrent le plus souvent un bruit de fond important et sont peu fiables d'un point de vue quantitatif : elles présentent une fenêtre de lecture réduite ne permettant pas un criblage optimal des molécules testées.

La demanderesse a au contraire élaboré un test de criblage hautement efficace basé sur la polarisation de fluorescence (Owicki J.C. et al., Journal of Biomolecular Screening, 5, 2000, 297-306). Cette technique permet par exemple de mesurer l'interaction entre un ligand marqué avec un fluorophore et un récepteur. Le principe consiste à mesurer une augmentation de la polarisation de fluorescence émise par le ligand fixé à son récepteur comparée à celle émise par le ligand libre. La polarisation de fluorescence du ligand libre est dépendante de son poids moléculaire et sera d'autant plus importante que le poids moléculaire dudit ligand sera élevé. Ainsi lorsque ce test est réalisé avec un ligand de fort poids moléculaire, ayant une forte polarisation de fluorescence intrinsèque, il sera difficile d'apprécier de façon fiable la différence de polarisation de fluorescence entre le ligand libre et le ligand fixé. L'utilisation d'un ligand de taille réduite permettra au contraire d'exacerber cette différence, et par conséquent d'augmenter la précision de l'essai. Il sera ainsi possible de mieux évaluer la réelle activité d'une molécule, et d'effectuer ces criblages à haut débit.

Plus particulièrement, le peptide comportant la séquence d'acides aminés décrites dans SEQ. ID. NO.1 ainsi que ses variants fonctionnels sont décrits.

Par « variants fonctionnels », on entend tous fragments ou mutants ponctuels du peptide décrit dans SEQ. ID. NO.1 capables d'interagir avec les protéines anti-apoptotiques de la famille Bcl-2 et plus particulièrement Bcl-2 et/ou Bcl-XL et/ou Bcl-W.

Ce peptide a été identifié par la méthode du double hybride en utilisant Bcl-XL, Bcl-W et Bcl-2 en tant que protéines « appâts ». Trois banques de cDNA humains (placenta, cerveau, lignée cellulaire CEMC7) ont été criblées, et ont permis l'identification de fragments « proies » correspondants à des séquences partielles de la séquence HC21ORF80 (Numéro d'Accession NM_015227).
Il a été ensuite déterminé expérimentalement par la technique du double hybride qu'un fragment de cette séquence est nécessaire et suffisant pour obtenir l'interaction avec Bcl-XL et/ou Bcl-2 et/ou Bcl-W, et correspond au fragment de 22 acides aminés décrit dans SEQ. ID. NO.1.

L'interaction avec les protéines Bcl-2, Bcl-W et Bcl-XL a été validée par des techniques biochimiques (co-immunoprécipitation, GST pull-down) et l'activité biologique de ce peptide a pu être confirmée par transfections et/ou microinjections dans des cellules où il a été montré qu'il induisait l'apoptose.

Les séquences d'acides nucléiques déduites selon le code génétique de la séquence d'acides aminés décrite dans SEQ. ID. NO.1 ainsi que de celles des variants fonctionnels décrits précédemment sont également décrits.

Plus particulièrement, l'invention concerne la séquence d'acide nucléique SEQ. ID. NO.2 codant pour le peptide décrit dans SEQ. ID. NO.1.

Par « séquences d'acides nucléiques », il doit être compris une séquence nucléique isolée de son contexte naturel. Il s'agit notamment de séquences isolées, amplifiées et/ou purifiées et éventuellement modifiées par génie génétique.

L'invention concerne aussi un vecteur recombinant comprenant une séquence d'acide nucléique selon l'invention.
Par « vecteur », il faut comprendre tout type de vecteur permettant l'introduction de la séquence d'acide nucléique dans une cellule-hôte et l'expression du polypeptide.
La vecteur recombinant selon l'invention est **caractérisé en ce qu**'il comprend les séquences d'ADN nécessaires à l'expression des peptides selon l'invention et plus particulièrement du peptide décrit dans SEQ. ID. NO.1.
On peut citer en particulier les vecteurs dérivés des plasmides bactériens, les bactériophages, les plasmides et chromosomes de levure, les virus...

L'invention porte aussi sur les cellules-hôtes transformées par les vecteurs recombinants. Ces cellules sont préférentiellement des bactéries ou des cellules eucaryotes. On peut citer à titre d'exemple *Escherichia coli*, les levures, les cellules d'insectes ou de mammifères.

L'invention porte par ailleurs sur un procédé de criblage d'agents capables de moduler l'interaction entre les peptides selon l'invention et plus particulièrement le peptide décrit dans SEQ. ID. NO.1, et des protéines anti-apoptotiques de la famille Bcl-2 et plus particulièrement Bcl-2, Bcl-W et Bcl-XL. Les agents modulateurs de ces interactions seront avantageusement des molécules synthétisées chimiquement ou issues de banques de produits.

Le procédé de criblage selon l'invention contient les étapes suivantes :
a) la préparation d'un peptide selon l'invention marqué avec un marqueur de fluorescence ;
b) l'incubation avec le composé à tester ;
c) l'ajout de la protéine de fusion contenant la protéine anti-apoptotique ;
d) la mesure de la polarisation de fluorescence.

L'invention concerne en particulier le procédé de criblage de molécules capables d'inhiber l'interaction entre le peptide et la protéine anti-apoptotique, comprenant les étapes suivantes :
a) la préparation du peptide selon l'invention marqué avec un marqueur de fluorescence ;
b) l'incubation ou non avec le composé à tester ;
c) l'ajout de la protéine de fusion contenant la protéine anti-apoptotique ;
d) la mesure de la polarisation de fluorescence avec et sans le composé à tester ;
e) la sélection des molécules pour lesquelles l'augmentation de la polarisation de fluorescence observée avec le composé à tester est significativement inférieure à celle observée sans le composé à tester.

L'invention concerne en particulier le procédé de criblage de molécules capables d'augmenter l'interaction entre le peptide et la protéine anti-apoptotique, comprenant les étapes suivantes :
a) la préparation du peptide selon l'invention marqué avec un marqueur de fluorescence ;
b) l'incubation ou non avec le composé à tester ;
c) l'ajout de la protéine de fusion contenant la protéine anti-apoptotique ;
d) la mesure de la polarisation de fluorescence avec et sans le composé à tester ;
e) la sélection des molécules pour lesquelles l'augmentation de la polarisation de fluorescence observée avec le composé à tester est significativement supérieure à celle observée sans le composé à tester.

Selon un mode de réalisation préféré des procédés précédemment décrits, le marqueur de fluorescence sera par exemple Oregon Green, Bodipy ou la fluorescéine, et plus particulièrement la fluorescéine.

Le peptide selon l'invention utilisé dans les procédés de criblage sera préférentiellement le peptide décrit dans SEQ. ID. NO.1.

Avantageusement, les procédés selon l'invention seront réalisés avec les protéines anti-apoptotiques de la famille Bcl-2 et plus particulièrement Bcl-2, Bcl-W et Bcl-XL.

Par conséquent, l'invention porte aussi sur l'utilisation des peptides selon l'invention pour le criblage selon les procédés de l'invention de molécules actives capables de moduler l'apoptose.

Plus particulièrement, l'invention concerne l'utilisation des peptides selon l'invention pour le criblage selon les procédés de l'invention de molécules utiles dans le traitement des pathologies impliquant une dérégulation de l'apoptose.

L'invention concerne donc l'utilisation des peptides selon l'invention pour le criblage selon les procédés de l'invention de molécules utiles dans le traitement des maladies autoimmunes, de certaines maladies neurologiques et des cancers.

### DESCRIPTION DES FIGURES

Figure 1 : "GST-Pulldown" GST-Bcl-XL + TBid avec compétition par le peptide décrit dans SEQ. ID. NO.1.
Figure 2 : Détermination du K_{d} du peptide décrit dans SEQ. ID. NO.1 pour Bcl-XL
Figure 3 : Détermination du K_{d} du peptide décrit dans SEQ. ID. NO.1 pour Bcl-W

Les exemples suivants illustrent l'invention et ne la limitent en aucune façon :

### EXEMPLE 1 : Identification du peptide décrit dans SEQ. ID. NO.1

Trois banques de cDNA humains ont été criblées (placenta, cerveau, lignée cellulaire CEMC7) par la technique du double hybride (Fields et col.) chez la levure en utilisant le protocole de conjugaison (Legrain et col., Nature Genetics, 1997, 16, 277-282).

### 1) Préparation des « appâts » et « proies »

a) Les « appâts » utilisés sont :
   - Bcl-XL délétée de son extrémité C-terminale (1-209) fusionnée au domaine de liaison à l'ADN LexA
   - Bcl-2 délétée de son extrémité C-terminale (1-211) fusionnée au domaine de liaison à l'ADN LexA.
   Ils sont exprimés dans *Saccharomyces cerevisiae* (CG1945 ou L40ΔGa14) et mis en préculture à 30°C dans un milieu synthétique dépourvu de tryptophane (DO-Trp) jusqu'à obtention d'une DO₆₀₀ₙₘ comprise entre 0,1 et 0,5. Cinquante ml d'une dilution de cette préculture (DO₆₀₀ₙₘ =0,006) sont incubés à 30°C pendant une nuit.
b) Une collection de levures contenant les plasmides exprimant les banques de cDNA fusionnés au domaine d'activation de la transcription Gal4 est obtenue par transformation après sélection sur un milieu dépourvu en leucine (DO-Leu). Ces levures sont aliquotées et conservées à -80°C.

### 2) Conjugaison

La conjugaison est réalisée avec un ratio « appât »/ « proie » égal à 2.
Une quantité de cellules de « levure-appâts » obtenues au stade 1)a) correspondant à 50 unités DO₆₀₀ₙₘ est mélangée aux « levure-proies » obtenues au stade 1)b). Après centrifugation, le culot est resuspendu dans un milieu YPGlu, étalé sur des boîtes de culture YPGlu et incubé 4 heures 30 à 30°C. La sélection des levures conjuguées contenant un « appât » et une « proie » capables d'interagir ensembles est réalisée sur un milieu DO-Leu-Trp-His : l'absence de leucine et de tryptophane permet de maintenir une pression de sélection ne permettant qu'aux levures contenant les deux types de plasmides (« appâts »/« proies ») de se développer ; l'absence d'histidine dans le milieu permet de sélectionner les levures conjuguées contenant un plasmide « appât » et un plasmide « proie » capables d'interagir ensembles : cette interaction permet d'activer le gène HIS3 codant pour une enzyme impliquée dans la biosynthèse de l'histidine.

### 3) Identification des clones positifs

Les fragments « proie » d'une colonie de levures sélectionnées selon 2) sont amplifiés par PCR à partir d'un lysat brut de cette colonie, en utilisant des amorces spécifiques du vecteur « proie » :
ABS1 5'-GCTTTGGAATCACTACAGG-3' (SEQ. ID. NO. 3),
ABS2 5'-CACGATGCACGTTGAAGTG-3' (SEQ. ID. NO. 4).
Les produits de PCR sont ensuite séquencés et les séquences obtenues sont identifiées par comparaison avec des banques de données.
Parmi les clones positifs obtenus, des fragments de 300 acides aminés environ ont pu être identifiés comme étant des séquences partielles de la séquence HC210RF80 (Numéro d'Accession : NM_015227).

### 4) Identification du peptide décrit dans SEQ. ID. NO. 1

Des expériences de double hybride réalisées selon les stades 1), 2) et 3) précédemment décrits à partir de plus petits fragments de la séquence HC21ORF80 ont permis d'identifier un petit peptide de 22 acides aminés comme étant nécessaire et suffisant pour obtenir l'interaction avec Bcl-XL et/ou Bcl-W et/ou Bcl-2 : Asp-Thr-Arg-Arg-Ser-Met-Val-Phe-Ala-Arg-His-Leu-Arg-Glu-Val-Gly-Asp-Glu-Phe-Arg-Ser-Arg (SEQ. ID. NO.1).

### EXEMPLE 2 : Validation de l'interaction entre le peptide décrit dans l'Exemple 1 et Bcl-W et/ou Bcl-XL et/ou Bcl-2

### 1) GST « pull-down»

L'interaction entre le peptide obtenu dans l'Exemple 1 et Bcl-W et/ou Bcl-XL et/ou Bcl-2 est validée par mesure du déplacement de l'interaction entre une protéine à motif « BH3 » Bid et la protéine de fusion GST-Bcl-XL, GST-Bcl-W ou GST-Bcl-2.

### a) Synthèse de Bid radiomarquée

La protéine marquée est obtenue en utilisant le kit TNT Quick Master (Promega). Quarante µl de mélange TNT sont incubés pendant 90 minutes à 30°C avec 2 µl (équivalent à 20 µCi) de ³⁵S-méthionine (Amersham), 1 µg d'ADN plasmidique codant pour Bid et de l'eau en quantité suffisante pour atteindre un volume de 50 µl.
Le nombre de fmoles/ µl de protéine radioactive produite est calculé à partir du nombre de méthionines dans la protéine.

### b) GST « pull-down »

Quatre fmoles de protéine Bid radioactive sont incubées à 4°C pendant 3 heures avec 3 µg de la protéine de fusion glutation-S-transférase-Bcl-XL (GST-Bcl-XL) ou glutation-S-transférase-Bcl-2 (GST-Bcl-2) ou glutation-S-transférase-Bcl-W (GST-Bcl-W) ou GST seule dans 300 µl de tampon de liaison (142 mM KCI, 5 mM MgCl₂, 10 mM tampon Hepes, 0,5 mM DTT, 1 mM EDTA, inhibiteur de protéases, pH 7,4) et 0,4% de Triton X100. Les billes de « glutathione sepharose 4 fast flow » (Amersham) sont lavées 3 fois dans le tampon de liaison et remises en suspension dans ce tampon de façon à obtenir une solution à 50%. 20 µl sont ajoutés à chaque échantillon et incubés sous rotation à 4 °C pendant 1 heure. Les billes sont ensuite lavées 3 fois dans le tampon de liaison, puis 25 µl de tampon 2x SDS, Laemmli (Sigma) sont ajoutés. Les échantillons sont placés 5 minutes à 95°C puis déposés sur un gel à 12% Tris-Glycine (Invitrogen). Après électrophorèse, le gel est ensuite incubé dans une solution de séchage (Invitrogen) pendant 30 minutes, puis séché pendant 150 minutes à 70°C. Les protéines radioactives sont révélées par exposition d'un film Kodak BioMax MS-1 (Sigma). Pour effectuer le test de compétition avec le peptide à tester, celui-ci est ajouté à la solution initiale avec des concentrations allant de 5 à 20 µM.

### c) Résultats

Lorsqu'on ajoute à la solution initiale le peptide obtenu dans l'Exemple 1, le signal autoradiographique de Bid disparaît. Ce résultat montre que le peptide obtenu dans l'Exemple 1 inhibe l'interaction entre Bcl-W et Bid, entre Bcl-XL et Bid et entre Bcl-2 et Bid.

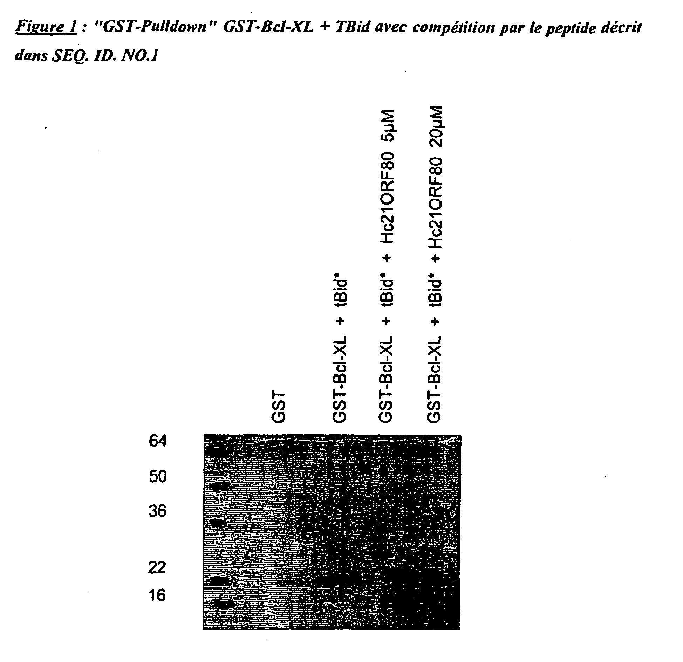

A titre d'exemple, le résultat obtenu entre Bcl-XL et Bid est rapporté dans la Figure 1.

### 2) Polarisation de fluorescence : Détermination du K_{d} de l'interaction entre le peptide obtenu dans l'Exemple 1 et la protéine antiapoptotique

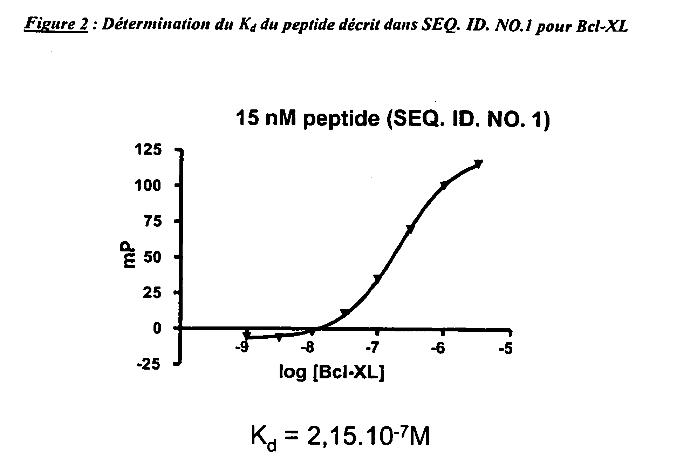

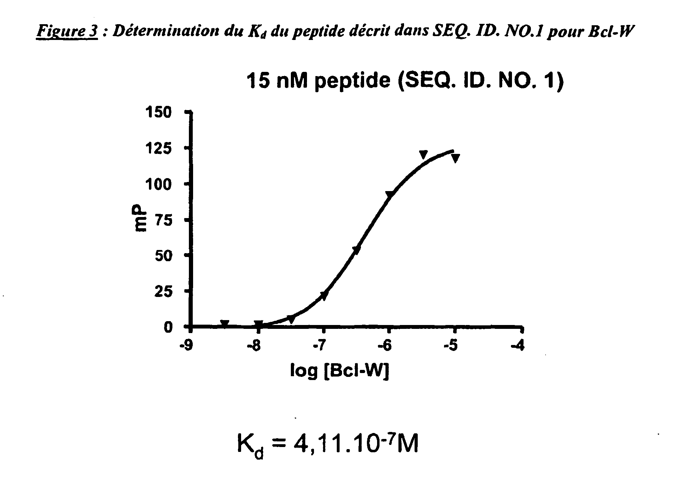

Une solution contenant 15 nM du peptide obtenu dans l'Exemple 1 marqué à la fluorescéine à l'extrémité N-terminale est mélangée à une solution contenant la protéine de fusion GST-Bcl-XL ou GST-Bcl-W à la concentration variant de 1 nM à 5 µM dans un tampon contenant Na₂HPO₄ 20 mM pH 7,4, de l'EDTA 1 mM, NaCl 50 mM, de l'acide pluronique F-68 0,05%. La polarisation de fluorescence est ensuite mesurée par l'appareil En Vision (Packard Perkin-Elmer).
Les résultats obtenus sont rapportés dans les Figures 2 et 3.

Résultats : Une augmentation significative de la polarisation de fluorescence est observée lorsque le peptide obtenu dans l'Exemple 1 est incubé avec les protéines de fusion contenant Bcl-XL et Bcl-W attestant de sa fixation sur ces protéines.

Le K_{d} obtenu avec Bcl-XL est de 2,15.10⁻⁷M (Figure 2) et celui obtenu avec Bcl-W est de 4,11.10⁻⁷M (Figure 3).

### EXEMPLE 3 : Test de criblage de molécules capables d'inhiber l'interaction entre Bcl-W et/ou Bcl-XL et/ou Bcl-2 et le peptide obtenu dans l'Exemple 1

Les produits à tester sont distribués dans des plaques 384 puits (Coming Flat Bottom) à une concentration finale de 10 µg/ml. Un puits est rempli avec une quantité équivalente de tampon/solvant sans composé à tester et constituera le témoin. Le peptide obtenu dans l'Exemple 1 marqué à la fluorescéine est ajouté dans chaque puits de manière à obtenir une concentration finale de 15 nM. La protéine de fusion GST-Bcl-XL, GST-Bcl-W ou GST-Bcl-2 est ensuite ajoutée de façon à obtenir une concentration finale de 500 nM (Bel-XL, Bcl-2) et de 1 µM (Bcl-W) dans un tampon contenant Na₂HPO₄ 20 mM pH 7,4, de l'EDTA 1 mM, NaCl 50 mM, de l'acide pluronique F-68 0,05%. La polarisation de fluorescence est ensuite mesurée par l'appareil En Vision (Packard Perkin-Elmer). Une diminution significative de la polarisation de fluorescence enregistrée dans l'essai réalisé avec le composé à tester comparée à celle obtenue sans le composé à tester (puits témoin) permet de conclure à une activité inhibitrice de la molécule. A l'inverse, une augmentation significative de la polarisation de fluorescence dans l'essai avec le produit à tester comparée au témoin permet de conclure à une activité activatrice de la molécule.

## Revendications

1. Peptide interagissant avec les protéines anti-apoptotiques de la famille Bcl-2 consistant en la séquence SEQ. ID. NO.1 : Asp-Thr-Arg-Arg-Ser-Met-Val-Phe-Ala-Arg-His-Leu-Arg-Glu-Val-Gly-Asp-Glu-Phe-Arg-Ser-Arg.

2. Peptide selon la revendication 1 interagissant avec les protéines anti-apoptotiques Bcl-2, Bcl-XL et/ou Bcl-W.

3. Peptide selon la revendication 1 ou 2 **caractérisé en ce qu'**il correspond à un fragment ou à un mutant ponctuel du peptide décrit dans SEQ, ID. NO.1.

4. Séquence d'acides nucléiques codant pour un peptide selon la revendication 1 consistant en la séquence SEQ. ID. NO.2:

5. Séquences d'acides nucléiques déduites selon le code génétique de la séquence d'acides aminés selon la revendication 1 ou 2.

6. Séquences d'acides nucléiques déduites selon le code génétique de la séquence d'acides aminés selon la revendication 3.

7. Vecteur recombinant **caractérisé en ce qu'**il comprend une séquence d'acides nucléiques selon l'une des revendications 4 à 6.

8. Vecteur recombinant selon la revendication 7 **caractérisé en ce que** le vecteur est un plasmide comprenant les séquences nécessaires à l'expression du peptide dans une cellule-hôte.

9. Cellule-hôte **caractérisée en ce qu'**elle est transformée par le vecteur recombinant selon l'une des revendications 7 ou 8.

10. Procédé d'identification de molécules capables de moduler l'interaction entre un peptide selon l'une des revendications 1, 2 ou 3 et une protéine anti-apoptotique de la famille Bcl-2, **caractérisé en ce qu'**il comprend les étapes suivantes :
a) la préparation d'un peptide selon l'une des revendications 1, 2 ou 3 marqué avec un marqueur de fluorescence ;
b) l'incubation avec le composé à tester ;
c) l'ajout de la protéine de fusion contenant la protéine anti-apoptotique de la famille Bcl-2 ;
d) la mesure de la polarisation de fluorescence.

11. Procédé d'identification de molécules capables d'inhiber l'interaction entre un peptide selon l'une des revendications 1, 2 ou 3 et une protéine anti-apoptotique de la famille Bcl-2, **caractérisé en ce qu'**il comprend les étapes suivantes :
a) la préparation d'un peptide selon l'une des revendications 1, 2 ou 3 marqué avec un marqueur de fluorescence ;
b) l'incubation avec ou sans le composé à tester ;
c) l'ajout de la protéine de fusion contenant la protéine anti-apoptotique de la famille Bcl-2 ;
d) la mesure de la polarisation de fluorescence ;
e) la sélection des molécules pour lesquelles l'augmentation de la polarisation de fluorescence observée avec le composé à tester est significativement inférieure à celle observée sans le composé à tester.

12. Procédé d'identification de molécules capables d'augmenter l'interaction entre un peptide selon l'une des revendications 1, 2 ou 3 et une protéine anti-apoptotique de la famille Bcl-2, **caractérisé en ce qu'**il comprend les étapes suivantes :
a) la préparation d'un peptide selon l'une des revendications 1, 2 ou 3 marqué avec un marqueur de fluorescence ;
b) l'incubation avec ou sans le composé à tester ;
c) l'ajout de la protéine de fusion contenant la protéine anti-apoptotique de la famille Bcl-2 ;
d) la mesure de la polarisation de fluorescence ;
e) la sélection des molécules pour lesquelles l'augmentation de la polarisation de fluorescence observée avec le composé à tester est significativement supérieure à celle observée sans le composé à tester.

13. Procédé selon l'une des revendications 10 à 12 dans lequel la protéine anti-apoptotique est Bcl-2.

14. Procédé selon l'une des revendications 10 à 12 dans lequel la protéine anti-apoptotique est Bcl-XL.

15. Procédé selon l'une des revendications 10 à 12 dans lequel la protéine anti-apoptotique est Bcl-W.

16. Procédé selon l'une des revendications 10 à 12 dans lequel le peptide utilisé est **caractérisé par** la séquence SEQ. ID. NO.1.

17. Procédé selon l'une des revendications 10 à 12 dans lequel le marqueur de fluorescence utilisé est la fluorescéine.

18. Utilisation d'un peptide selon l'une des revendications 1, 2 ou 3 pour l'identification selon le procédé d'une des revendications 10 à 17 de molécules modulatrices de l'apoptose.

19. Utilisation d'un peptide selon l'une des revendications 1, 2 ou 3 pour l'identification selon le procédé d'une des revendications 10 à 17 de molécules utiles dans le traitement des pathologies impliquant une dérégulation de l'apoptose.

20. Utilisation d'un peptide selon l'une des revendications 1, 2 ou 3 pour l'identification selon le procédé d'une des revendications 10 à 17 de molécules utiles dans le traitement des maladies autoimmunes, de certaines maladies neurologiques et des cancers.

## Claims

1. Peptide interacting with anti-apoptotic proteins of the Bcl-2 family consisting of the sequence SEQ. ID. NO.1 : Asp-Thr-Arg-Arg-Ser-Met-Val-Phe-Ala-Arg-His-Leu-Arg-Glu-Val-Gly-Asp-Glu-Phe-Arg-Ser-Arg.

2. Peptide according to claim 1 interacting with the anti-apoptotic proteins Bcl-2, Bcl-XL and/or Bcl-W.

3. Peptide according to claim 1 or 2, **characterised in that** it corresponds to a fragment or point mutant of the peptide described in SEQ. ID. NO.1.

4. Nucleic acid sequence coding for a peptide according to claim 1 consisting of the sequence SEQ. ID. NO.2 :

5. Nucleic acid sequences deduced according to the genetic code from the amino acid sequence according to claim 1 or 2.

6. Nucleic acid sequences deduced according to the genetic code from the amino acid sequence according to claim 3.

7. Recombinant vector, **characterised in that** it comprises a nucleic acid sequence according to one of claims 4 to 6.

8. Recombinant vector according to claim 7, **characterised in that** the vector is a plasmid comprising the sequences necessary for expression of the peptide in a host cell.

9. Host cell, **characterised in that** it has been transformed by the recombinant vector according to one of claims 7 or 8.

10. Method of identifying compounds capable of modifying the interaction between a peptide according to one of claims 1, 2 or 3 and an anti-apoptotic protein of the Bcl-2 family, **characterised in that** it comprises the following steps:
a) preparation of a peptide according to one of claims 1, 2 or 3 labelled with a fluorescent label;
b) incubation with the compound under test;
c) addition of the fusion protein comprising the anti-apoptotic protein of the Bcl-2 family;
d) measurement of the fluorescence polarisation.

11. Method of identifying compounds capable of inhibiting the interaction between a peptide according to one of claims 1, 2 or 3 and an anti-apoptotic protein of the Bcl-2 family, **characterised in that** it comprises the following steps:
a) preparation of a peptide according to one of claims 1, 2 or 3 labelled with a fluorescent label;
b) incubation with or without the compound under test;
c) addition of the fusion protein comprising the anti-apoptotic protein of the Bcl-2 family;
d) measurement of the fluorescence polarisation;
e) selection of the compounds for which the increase in fluorescence polarisation observed with the compound under test is significantly less than that observed without the compound under test.

12. Method of identifying compounds capable of enhancing the interaction between a peptide according to one of claims 1, 2 or 3 and an anti-apoptotic protein of the Bcl-2 family, **characterised in that** it comprises the following steps:
a) preparation of a peptide according to one of claims 1, 2 or 3 labelled with a fluorescent label;
b) incubation with or without the compound under test;
c) addition of the fusion protein comprising the anti-apoptotic protein of the Bcl-2 family;
d) measurement of the fluorescence polarisation;
e) selection of the compounds for which the increase in fluorescence polarisation observed with the compound under test is significantly greater than that observed without the compound under test.

13. Method according to one of claims 10 to 12, wherein the anti-apoptotic protein is Bcl-2.

14. Method according to one of claims 10 to 12, wherein the anti-apoptotic protein is Bcl-XL.

15. Method according to one of claims 10 to 12, wherein the anti-apoptotic protein is Bcl-W.

16. Method according to one of claims 10 to 12, wherein the peptide used is **characterised by** the sequence SEQ. ID. NO.1.

17. Method according to one of claims 10 to 12, wherein the fluorescent label used is fluorescein.

18. Use of a peptide according to one of claims 1, 2 or 3 in the identification, according to the method of one of claims 10 to 17, of apoptosis-modifying compounds.

19. Use of a peptide according to one of claims 1, 2 or 3 in the identification, according to the method of one of claims 10 to 17, of compounds that are useful in the treatment of pathologies involving deregulation of apoptosis.

20. Use of a peptide according to one of claims 1, 2 or 3 in the identification, according to the method of one of claims 10 to 17, of compounds that are useful in the treatment of autoimmune diseases, certain neurological disorders and cancers.

## Patentansprüche

1. Peptid, welches mit den anti-apoptotischen Proteinen der Familie Bcl-2 interagiert, bestehend aus der Sequenz SEQ. ID. NO.1 : Asp-Thr-Arg-Arg-Ser-Met-Val-Phe-Ala-Arg-His-Leu-Arg-Glu-Val-Gly-Asp-Glu-Phe-Arg-Ser-Arg.

2. Peptid nach Anspruch 1, welches mit den anti-apoptotischen Proteinen Bcl-2, Bcl-XL und/oder Bcl-W interagiert.

3. Peptid nach Anspruch 1 oder 2, **dadurch gekennzeichnet, dass** es einem Fragment oder einer Punktmutante des in SEQ. ID. NO.1 beschriebenen Peptids entspricht.

4. Nucleinsäuresequenz codierend für ein Peptid nach Anspruch 1, bestehend aus der Sequenz SEQ. ID. NO. 2:

5. Nucleinsäuresequenzen abgeleitet von dem genetischen Code der Aminosäuresequenz nach Anspruch 1 oder 2.

6. Nucleinsäuresequenzen abgeleitet nach dem genetischen Code der Aminosäuresequenz nach Anspruch 3.

7. Rekombinanter Vektor, **dadurch gekennzeichnet, dass** er eine Nucleinsäuresequenz nach einem der Ansprüche 4 bis 6 umfasst.

8. Rekombinanter Vektor nach Anspruch 7, **dadurch gekennzeichnet, dass** der Vektor ein Plasmid ist, welches die notwendigen Sequenzen für die Expression des Peptids in einer Wirtszelle umfasst.

9. Wirtszelle, **dadurch gekennzeichnet, dass** sie durch den rekombinanten Vektor nach einem der Ansprüche 7 oder 8 transformiert ist.

10. Verfahren zur Identifizierung von Molekülen, die dazu in der Lage sind, die Wechselwirkung zwischen einem Peptid nach einem der Ansprüche 1, 2 oder 3 und einem anti-apoptotischen Protein der Familie Bcl-2 zu modulieren, **dadurch gekennzeichnet, dass** es die folgenden Stufen umfasst:
a) Herstellung eines Peptids nach einem der Ansprüche 1, 2 oder 3, welches mit einem Fluoreszenzmarker markiert ist;
b) Inkubierung mit der zu untersuchtenden Verbindung;
c) Zugabe des Fusionsproteins, welches das anti-apoptotische Protein der Familie Bcl-2 enthält;
d) Messung der Fluoreszenzpolarisation.

11. Verfahren zur Identifizierung von Molekülen, die dazu in der Lage sind, die Wechselwirkung zwischen einem Peptid gemäß einem der Ansprüche 1, 2 oder 3 und einem anti-apoptotischen Protein der Familie Bcl-2 zu inhibieren, **dadurch gekennzeichnet, dass** es die folgenden Stufen umfasst:
a) Herstellung eines Peptids nach einem der Ansprüche 1, 2 oder 3, welches mit einem Fluoreszenzmarker markiert ist;
b) Inkubierung mit oder ohne die zu untersuchende Verbindung;
c) Zugabe des Fusionsproteins, welches das anti-apoptotische Protein der Familie Bcl-2 enthält;
d) Messung der Fluoreszenzpolarisation;
e) Selektion der Moleküle, bei denen die mit der zu untersuchenden Verbindung beobachtete Erhöhung der Fluoreszenzpolarisation signifikant geringer ist als jene, die man ohne die zu untersuchende Verbindung beobachtet.

12. Verfahren zur Identifizierung von Molekülen, die dazu in der Lage sind, die Wechselwirkung zwischen einem Peptid nach einem der Ansprüche 1, 2 oder 3 und einem anti-apoptotischen Protein der Familie Bcl-2 zu steigern, **dadurch gekennzeichnet, dass** es die folgenden Stufen umfasst:
a) Herstellung eines Peptids nach einem der Ansprüche 1, 2 oder 3, welches mit einem Fluoreszenzmarker markiert ist;
b) Inkubierung mit oder ohne die zu untersuchende Verbindung;
c) Zugabe des Fusionsproteins, welches das anti-apoptotische Protein der Familie Bcl-2 enthält;
d) Messung der Fluoreszenzpolarisation;
e) Selektion der Moleküle, bei denen die mit der zu untersuchenden Verbindung beobachtete Erhöhung der Fluoreszenzpolarisation signifikant größer ist als jene, die man mit der zu untersuchenden Verbindung beobachtet.

13. Verfahren nach einem der Ansprüche 10 bis 12, worin das anti-apoptotische Protein Bcl-2 ist.

14. Verfahren nach einem der Ansprüche 10 bis 12, worin das anti-apoptotische Protein Bcl-XL ist.

15. Verfahren nach einem der Ansprüche 10 bis 12, worin das anti-apoptotische Protein Bcl-W ist.

16. Verfahren nach einem der Ansprüche 10 bis 12, worin das verwendete Peptid durch die Sequenz SEQ. ID. NO.1 **gekennzeichnet** ist.

17. Verfahren nach einem der Ansprüche 10 bis 12, worin der verwendete Fluoreszenzmarker Fluorescein ist.

18. Verwendung eines Peptids nach einem der Ansprüche 1, 2 oder 3 für die Identifizierung nach dem Verfahren nach einem der Ansprüche 10 bis 17 von die Apoptose modulierenden Molekülen.

19. Verwendung eines Peptids nach einem der Ansprüche 1, 2 oder 3 für die Identifizierung nach dem Verfahren gemäß einem der Ansprüche 10 bis 17 von Molekülen, die nützlich sind bei der Behandlung von Erkrankungen, bei denen eine Deregulierung der Apoptose beteiligt ist.

20. Verwendung eines Peptids nach einem der Ansprüche 1, 2 oder 3 für die Identifizierung nach dem Verfahren gemäß einem der Ansprüche 10 bis 17 von Molekülen, die nützlich sind bei der Behandlung von Autoimmunerkrankungen, bestimmten Nervenerkrankungen und Krebs.


### LISTAGE DES SEQUENCES

<110> LES LABORATOIRES SERVIER HYBRIGENICS
<120> Nouveau peptide interagissant avec les protéines antiapoptotiques de la famille Bcl-2
<130> Peptide-1
<150> FR 03.09697
   <151> 2003-08-06
<160> 4
<170> PatentIn version 3.1
<210> 1
   <211> 22
   <212> PRT
   <213> Homo sapiens
<400> 1
   Asp Thr Arg Arg Ser Met Val Phe Ala Arg His Leu Arg Glu Val Gly
   1 5 10 15
   Asp Glu Phe Arg Ser Arg
   20
<210> 2
   <211> 66
   <212> DNA
   <213> Homo sapiens
<400> 2
   gatacccgtc gcagcatggt gtttgccagg cacctgcggg aggtgggaga cgagttcagg
   60
   agcaga
   66
<210> 3
   <211> 19
   <212> DNA
   <213> Artificial
<400> 3
   gctttggaat cactacagg
   19
<210> 4
   <211> 19
   <212> DNA
   <213> Artificial
<400> 4
   cacgatgcac gttgaagtg
   19